# EUROPEAN PATENT APPLICATION

(11) **EP 2 161 030 A1**
(43) Date of publication of application: **10.03.2010**
(21) Application number: 08163932.0
(22) Date of filing: 09.09.2008
(51) Int. Cl.: A61K 38/11

(54) **Oxytocin formulations and uses thereof**

(71) Applicant: Rijksuniversiteit te Groningen, 9712 CP Groningen (NL)
(72) Inventor: The designation of the inventor has not yet been filed
(74) Representative: Hatzmann, Martin

(57) **Abstract**

The present invention relates to the field of preventive and therapeutic medicine, in particular, oxytocin formulations. Provided is an aqueous oxytocin formulation comprising oxytocin or a functional analogue thereof, and a non-toxic source of metal ions, and the use of the formulation for the manufacture of a medicament for the therapeutic and/or prophylactic treatment of haemorrhage. Also, provided is a method for treating or preventing haemorrhage in a subject in need thereof, comprising administering to said subject an effective dosage amount of a formulation according to the invention.

## Description

The present invention relates to the field of preventive and therapeutic medicine. In particular, it relates to oxytocin formulations and uses thereof in indications such as, but not limited to, induction of labor, augmentation of labor, post-partum haemorrhage or uterine atony.
Oxytocin and functional analogues thereof are widely used in human and veterinarian medicine to induce labor and support labor in case of non-progression of parturition and to treat (post-partum) haemorrhage. Currently, oxytocin is considered to be the principal agent to treat post-partum haemorrhage. The nonapeptide oxytocin is a mammalian hormone. It is degraded in the gastrointestinal tract, therefore it is administered as liquid formulation by injection or as nasal spray. Its half-life in the blood is typically about three minutes. Synthetic oxytocin is commercially available as ready-to-use liquid formulations under the trade names Pitocin® and Syntocinon® or as generic oxytocin. Functional oxytocin analogues are known in the art, such as desamino-oxytocin (Hope et al., J Biol Chem 237 (5): 1563-1566, 1962) and carbetocin (Sweeney et al, Curr Ther Res 47: 528-540, 1990). Both desamino-oxytocin and carbetocin (trade name Duratocin®) are commercially available.
According to the World Health Report 2005 as issued by the World Health Organization, in Africa, Asia and Latin America each year half a million women die as a result of problems during pregnancy and childbirth. In Africa and Asia, at least 25% of those deaths can be attributed to haemorrhage, most commonly caused by failure of the uterus to contract adequately after childbirth (atonicity). This makes haemorrhage the leading cause of maternal deaths in these continents (Khan et al., Lancet 367 (9516): 1066-1074, 2006). In third world countries, it is often practically and/or economically impossible to protect pharmaceutical preparations from the harmful effects of high temperatures during transportation, storage and use. Reports and stability studies of injectable oxytocins have shown serious instability on exposure to increased temperatures and exposure to light (see de Groot et al., World Health Organization, WHO/DAP/94.13, 1994 and de Groot et al., J Clin Pharm Ther 20 (2): 115-119, 2008, and references cited therein). The 1994 publication by de Groot *et al*. discloses that tablets of oral oxytocins (ergometrine, methylergometyrine, oxytocin and desamino-oxytocin) are not stable under simulated tropical conditions, and concludes that oral oxytocins are not suitable for use in the prevention of postpartum heamorrhage. Thus, despite the availability of various formulations of oxytocin and desamino-oxytocin for treatment of haemorrhage, the (sub)tropical ambient temperatures and the absence of a so-called cold-chain in these parts of the world severely affect their stability and functionality. Proper prophylaxis and / or treatment of haemorrhage is therefore nearly impossible, resulting in this astonishingly high number of casualties each year.

Given the reduced stability of oxytocin formulations at increased temperatures, their use in treatment methods against (post-partum) haemorrhage in (sub)tropical countries, including many third world countries, is limited. This causes a high incidence of maternal deaths. Thus, there is a clear need for oxytocin formulations, preferably as a ready-to-use injectable aqueous solution, that has an improved thermal stability.

It is therefore an object of the invention to provide means and methods to increase the thermal stability of aqueous, oxytocin-formulations. A further object is the provision of a therapeutic or prophylactic oxytocin-formulation against haemorrhage, e.g. a ready-to-use medication that can be administered to a subject in need thereof, that has a stability that can withstand (sub)tropical ambient temperatures, is cheap to produce and / or is free of side effects. In a specific aspect, the invention aims to provide an injectable oxytocin formulation displaying improved thermal stability as compared to existing formulations

The present inventors surprisingly discovered that the stability of aqueous oxytocin formulations is greatly enhanced by the addition of non-toxic metal ions. For example, aqueous oxytocin formulations show much less degradation when kept at 55 °C for at least 4 weeks when formulated with metal ions. The invention accordingly relates to the use of non-toxic metal ions for stabilizing liquid oxytocin formulations comprising oxytocin or a functional analogue thereof. Furthermore, it relates to formulations and methods for the prophylaxis or treatment of haemorrhage in a subject in need thereof. Provided is an aqueous oxytocin formulation comprising oxytocin or a functional analogue thereof, and a non-toxic (i.e. biocompatible) source of metal ions. Also provided is the use of the formulation for the manufacture of a medicament for the therapeutic and/or prophylactic treatment of haemorrhage. The invention also provides a method for treating or preventing haemorrhage in a subject in need thereof, comprising administering to said subject an effective dosage amount of a formulation according to the invention.

In one aspect, the invention provides an aqueous oxytocin formulation comprising oxytocin or a functional analogue thereof, and a non-toxic source of metal cations. The source of these metal ions may be a metal salt. Preferably, the source is a metal chloride salt. The metal ions may be monovalent metal cations, for example Na⁺ or K⁺, used at non-toxic concentrations and amounts or divalent metal ions, for instance selected from the group consisting of Ca²⁺, Mg²⁺, Cu²⁺ and Zn²⁺ used at non-toxic concentrations and amounts. A preferred divalent metal ion is Ca²⁺. Any type of biocompatible metal chloride salt may be used as metal ion source. Useful metal chloride salts include NaCl, KCl, CaCl₂, MgCl₂, CuCl₂ and ZnCl₂. Preferably, NaCl and/or CaCl₂ are used. In one embodiment, the invention provides an aqueous oxytocin formulation comprising oxytocin or a functional analogue thereof, and at least one monovalent metal ion and at least one divalent metal ion. For example, a formulation may comprise oxytocin, Na⁺ ions and Ca²⁺ ions. In a specific embodiment, the oxytocin formulation comprises oxytocin or a functional analogue thereof, and at least one monovalent metal chloride salt and/or at least one bivalent metal chloride salt. For example, a formulation comprises oxytocin, NaCl, ZnCl₂ and/or CaCl₂.
As shown herein below, the stabilizing effect of metal ions in an aqueous oxytocin formulation was determined by measuring the recovery of oxytocin (remaining oxytocin as % of initial amount), the amount of oxytocin-monomers (% of total remaining oxytocin) and / or the level of aggregation (aggregated oxytocin as % of total remaining oxytocin) upon prolonged storage at elevated (e.g. 55°C) temperatures. These parameters can be assessed by using any method for analysis deemed suitable by a person skilled in the art. Examples of suitable methods for analysis include, but are not limited to, reversed-phase high-performance liquid chromatography (RP-HPLC) and high pressure size exclusion chromatography (HP-SEC). Thus, suitable concentrations of metal ions to achieve the stabilizing effect at elevated temperatures can be determined by those skilled in the art. They may vary, e.g. depending on the composition of the formulation or its intended application. In one embodiment, a formulation comprises oxytocin and at least 50 nM, preferably at least 0.5 mM, more preferably at least 5 mM metal ions. In another embodiment, a formulation comprises oxytocin and metal ions in a concentration of up to 1 M, preferably up to 500 mM, more preferably up to 200 mM. In one embodiment, between 0.5 and 150 mM metal ions, preferably 5-100 mM, such as 10, 20, 25, 30, 40 or 50 mM, are used to stabilize aqueous oxytocin-formulations. In another embodiment, a metal ion concentration in the range from 20 to 200 mM is used, like 30-180 mM, 40- 150 mM or 50-100 mM. Other suitable ranges include between 1 and 50 mM, between 30 and 60 mM and between 40 and 70 mM metal ions are used.

For example, a formulation may comprise oxytocin and 10 mM Zn²⁺. A formulation may, as another example, comprise oxytocin, 20 mM NaCl and 10 mM CaCl₂. For example, a formulation may comprise oxytocin and 200 mM NaCl. A formulation may, for another example, comprise oxytocin, 50 mM MgCl₂ and 50 mM CaCl₂. In one embodiment, a formulation comprises oxytocin and between 1 and 50 mM MgCl₂. In another embodiment, a formulation comprises oxytocin and between 30 and 80 mM metal ions, such as CaCl₂. In another embodiment, a formulation comprises oxytocin and between 60 and 400 mM metal ions. For example, a formulation may comprise 200 mM NaCl. In yet another embodiment, a formulation comprises oxytocin and between 300 and 800 mM metal ions. In a preferred embodiment, a formulation comprises oxytocin and between 5 and 100 mM metal ions.

In another aspect, the invention provides an aqueous oxytocin formulation comprising oxytocin or a functional analogue thereof, and a non-toxic source of chloride anions. The source of chloride ions may be a chloride salt, preferably a metal chloride salt. Suitable concentrations of chloride anions can be determined by those skilled in the art and may vary depending on the composition of the formulation or its intended application. In one embodiment, a formulation according to the invention comprises oxytocin and at least 0.05 mM chloride anions, preferably at least 0.5 mM, more preferably at least 5 mM. A formulation may comprise oxytocin and up to 1 M chloride anions, preferably up to 500 mM chloride anions, more preferably up to 100 mM chloride anions. In one embodiment, a formulation comprises oxytocin and between 2 and 200 mM chloride anions, preferably between 5 and 100 mM chloride anions. In another embodiment, a formulation comprises between 50 and 400 mM chloride anions.

According to another object of the invention, the invention provides the use of non-toxic metal ions and/or chloride anions to stabilize a liquid formulation of oxytocin or a functional analogue thereof. The source(s) and concentrations of these metal ions which may be used are described herein above. Preferably, the source is a metal chloride salt.

As will be understood, a formulation as provided herein comprises a therapeutically effective amount of oxytocin or a functional analogue thereof. In one aspect, the invention provides a formulation comprising at least 5 mIU/ml, more preferably at least 50 mIU/ml, more preferably at least 500 mIU/ml oxytocin or a functional analogue thereof, and metal ions. In another aspect, the invention provides a formulation comprising up to 1000 IU/ml, more preferably up to 500 IU/ml, more preferably up to 100 IU/ml oxytocin or a functional analogue thereof, and metal ions. In one embodiment, a formulation comprises at least 500 mIU/ml oxytocin and, preferably at least 5 mM, metal ions such as CaCl₂. In another embodiment, a formulation comprises between 5 and 300 IU/ml oxytocin and, preferably between 1 and 300 mM, metal ions. In another embodiment, a formulation comprises between 1 and 60 IU/ml oxytocin and, preferably between 10 and 200 mM, metal ions such as Mg²⁺. In yet another embodiment, a formulation comprises between 10 and 200 IU/ml oxytocin and, preferably between 50 and 300 mM, metal ions. In a preferred embodiment, a formulation comprises between 5 and 100 IU/ml oxytocin and, preferably between 5 and 100 mM, metal ions such as CaCl₂.

The stability of a formulation according to the invention can be further enhanced by addition of a buffer salt, to maintain a stable pH. Useful buffers include phosphate buffer, acetate buffer, aspartate buffer and citric acid buffer. In one embodiment the invention provides a formulation comprising oxytocin or a functional analogue thereof and a source of non-toxic metal ions, further comprising a buffer selected from the group consisting of phosphate buffer, acetate buffer, aspartate buffer and citric acid buffer. In one embodiment, the invention provides a formulation comprising oxytocin or a functional analogue thereof, at least 0.05 mM metal ions, preferably at least 0.5 mM metal ions, more preferably at least 5 mM metal ions and between 1 and 100 mM acetate buffer. For example, a formulation may comprise oxytocin, 10 mM Mg²⁺ and 10 mM acetate buffer. In another embodiment, the invention provides a formulation comprising oxytocin or a functional analogue thereof, between 5 and 200 mM metal ions and between 1 and 100 mM aspartate buffer. For example, a formulation comprises oxytocin, 50 mM CaCl₂ and 50 mM aspartate buffer. Good results were observed after addition of citric acid buffer. Therefore, in a preferred embodiment the invention provides a formulation comprising oxytocin or a functional analogue thereof, e.g. in a concentration of up to 50 IU/ml, metal ions, preferably up to 100 mM, and citric acid buffer.
Good stability was observed under slightly acidic pH. The invention therefore provides a formulation comprising oxytocin or a functional analogue thereof and a source of non-toxic metal ions, further comprising a buffer and having a pH between 3 and 6, preferably between 3 and 5, more preferably between 3.8 and 4.8. In one embodiment, a formulation comprises between 5 and 60 IU/ml oxytocin, between 50 and 200 mM metal ions and citric acid buffer while having a pH of between 4 and 5. In another embodiment, a formulation comprises between 30 and 200 IU/ml oxytocin, between 80 and 300 mM metal ions and phosphate buffer while having a pH of between 4 and 5. In a preferred embodiment, a formulation comprises between 5 and 100 IU/ml oxytocin, between 5 and 100 mM metal ions and citric acid buffer while having a pH of 4.0 to 4.5. For example, a formulation may comprise 10 IU/ml oxytocin, 20 mM MgCl₂ and 10 mM citric acid buffer.

Very good stability was observed in the presence of CaCl₂ and citric acid buffer. In one embodiment, the invention therefore provides a formulation comprising oxytocin or a functional analogue thereof, CaCl₂ and citric acid. For example, it contains between 0.1 and 60 mM CaCl₂ and between 5 and 200 mM citric acid buffer. In another embodiment, the formulation comprises between 50 and 200 mM CaCl₂ and between 1 and 100 mM citric acid buffer. For example, a formulation comprises oxytocin, e.g. in a concentration of up to 50 IU/ml, 50 mM CaCl₂ and 50 mM citric acid buffer. In yet another embodiment, a formulation comprises between 1 and 100 IU/ml oxytocin, between 30 and 300 mM CaCl₂ and between 30 and 500 mM citric acid buffer. In a preferred embodiment, a formulation comprises between 5 and 100 IU/ml oxytocin, between 5 and 100 mM CaCl₂ and between 1 and 100 mM citric acid buffer. For example, a formulation may comprise oxytocin, e.g. in a concentration of up to 50 IU/ml, 10 mM CaCl₂ and 10 mM citric acid buffer.

The invention also provides an aqueous oxytocin formulation comprising oxytocin or a functional analogue thereof and a non-toxic source of metal ions, for use as a medication. Also provided is a package or spray container comprising a formulation according to the invention. In view of the light sensitivity of oxytocin injectables, a package is preferably light resistant. Exemplary packages include vials, ampoules, plastic containers or a sprayer. The package or spray container may contain instructions for use.

According to another aspect of the invention, the invention provides the use of aqueous oxytocin formulations comprising oxytocin or a functional analogue thereof and a non-toxic source of metal ions, for the manufacture of a medicament for the therapeutic and/or prophylactic treatment of haemorrhage, such as post-partum haemorrhage. These medicaments may be administered to a subject in need thereof by any means known to be suitable to those skilled in the art, including intravenous, subcutaneous, intramuscular, and mucosal administration.
A formulation may thus be formulated for administration via the intravenous, subcutaneous, intramuscular, and mucosal route. A formulation is preferably administered via the subcutaneous route, the mucosal route, or a combination thereof. The mucosal route may be exemplified by, but is not limited to, the pulmonary, nasal, sublingual or buccal route. Accordingly, in one embodiment the invention provides the use of formulations according to the invention, for the manufacture of a medicament formulated for subcutaneous or mucosal administration, or a combination thereof.
For intravenous, subcutaneous or intramuscular administration, the formulation may be provided as a sterile solution, suspension or an emulsion. The formulation may be applied by means of an injection or an infusion. For mucosal administration, the formulation may be provided as an aqueous spray and may be applied directly by means of a spray container or an inhalator. Alternatively, but not limited to, the oxytocin formulation may be administered to the mucosa as an aqueous gel.

According to another aspect of the invention, the invention provides a method for treating or preventing haemorrhage in a subject in need thereof, comprising administering to said subject an effective dosage amount of an oxytocin formulation according to the invention. The subject may be living under (sub)tropical conditions. The haemorrhage may be post-partum haemorrhage. The effective dosage amount according to methods of the invention may be administered via the intravenous, subcutaneous or intramuscular route, the mucosal route, or a combination thereof. As mentioned supra, the mucosal route may be exemplified by, but is not limited to, the pulmonary, nasal, sublingual or buccal route.

Effective amounts of oxytocin or a functional analogue thereof are amounts that are sufficient to bring about an efficacious effect against symptoms associated with haemorrhage, and can be determined by those skilled in the art by routine experimentation. Furthermore, professional guidelines on the use of oxytocin in various indications exist and its use has been described in handbooks (e.g. AHFS Drug Information or Martindale, The Extra Pharmacopoeia). In general, an effective dosage of oxytocin for the purposes of this invention is expected to comprise a bolus amount of between 2 and 10 IU. Thereafter, 5-10 IU or, in serious cases, up to 20 IU of oxytocin may be administered by infusion. In one aspect the invention provides a method for treating or preventing haemorrhage in a subject in need thereof, comprising administering to said subject a bolus amount of up to 2 IU of oxytocin or a functional analogue thereof, preferably up to 5 IU, more preferably up to 10 IU. In another aspect the invention provides a method for treating or preventing haemorrhage in a subject in need thereof, comprising administering to said subject a bolus amount of up to 2 IU, preferably up to 5 IU, more preferably up to 10 IU of oxytocin or a functional analogue thereof, further comprising an infusion of up to 20 IU, preferably up to 10 IU of oxytocin or a functional analogue thereof.
The invention is exemplified by the following examples.

### LEGENDS TO THE FIGURES

Figure 1: Recovery of oxytocin (OT) in the presence of 60 mM phosphate buffer with or without 150 mM NaCl, stored for 4 days at pH 4.5 and a temperature of 60 °C. Recovery is expressed as the percentage of oxytocin that is recovered upon storage at 4°C for the same period of time.
Figure 2: Recovery of oxytocin in an aqueous formulation comprising 10 mM acetate buffer in the presence of 0, 10 or 50 mM Ca²⁺, Mg²⁺ or Zn²⁺ ions (as chloride salts), upon storage for 4 weeks at pH 4.5 (10 mM acetate-buffer) and at a temperature of 55 °C. Panel A: recovery determined by RP-HPLC. Panel B: oxytocin monomer recovery determined by HP-SEC.
Figure 3: Recovery of oxytocin (determined by RP-HPLC or HP-SEC) in the absence or presence of different metal salts after 4 weeks-storage at 55 °C in 10 mM acetate buffer.
Figure 4: Oxytocin recovery in the presence of 0, 10 or 50 mM CaCl₂ and 10 or 50 mM buffer. Cit = citric acid buffer, Ac = acetate buffer, Asp = aspartate buffer. Panel A: recovery after 4 weeks-storage at 55 °C determined by RP-HPLC. Panel B: oxytocin aggregate recovery after 4 weeks storage at a temperature of 55 °C determined by HP-SEC.
Figure 5: Oxytocin-recovery after 4 weeks-storage at 55 °C in the presence of 0, 10 or 50 mM CaCl₂ and different buffers. Cit = citric acid buffer, Acet = acetate buffer, Asp = aspartate buffer.
Figure 6: Oxytocin (OT)-recovery after 4 days-storage at 60 °C in the presence of CaCl₂ and citric acid buffer. Panel A: recovery as determined by RP-HPLC. Panel B: Percentage oxytocin monomers as determined by HP-SEC. Panel C: Percentage oxytocin aggregates as determined by HP-SEC.

### EXAMPLE 1

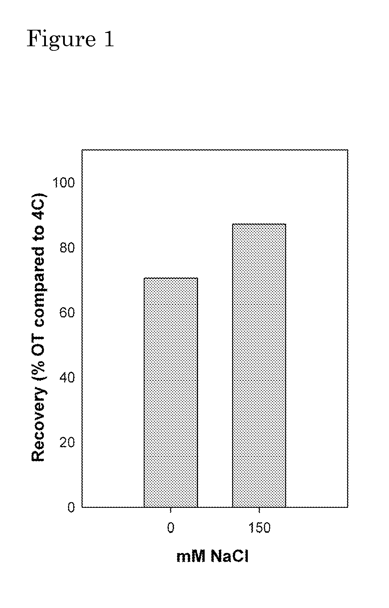

This example demonstrates the oxytocin-stabilizing effect of metal ions. Metal chloride salt NaCl (150 mM) was added to a formulation comprising oxytocin and 60 mM phosphate buffer, pH 4.5. Samples of the formulation were then stored for 4 days at 4 °C (reference control) or 60 °C (test). Thereafter, all samples were stored under cooled conditions (2-8 °C) before analysis by RP-HPLC. Effects on stability were determined by measuring the recovery of oxytocin (i.e. the remaining percentage of oxytocin after incubation at 60 °C compared to the remaining percentage of oxytocin after incubation at 4 °C). The results are presented in Figure 1.

Figure 1 shows oxytocin-stability in phosphate buffer in the absence of metal ions after 4 days-storage at 60 °C, as expressed by recovery of oxytocin relative to the sample stored at 4°C. Storage at 60 °C resulted in a loss of almost 30%. In contrast, in the presence of NaCl, oxytocin recovery was approximately 85%.

### EXAMPLE 2

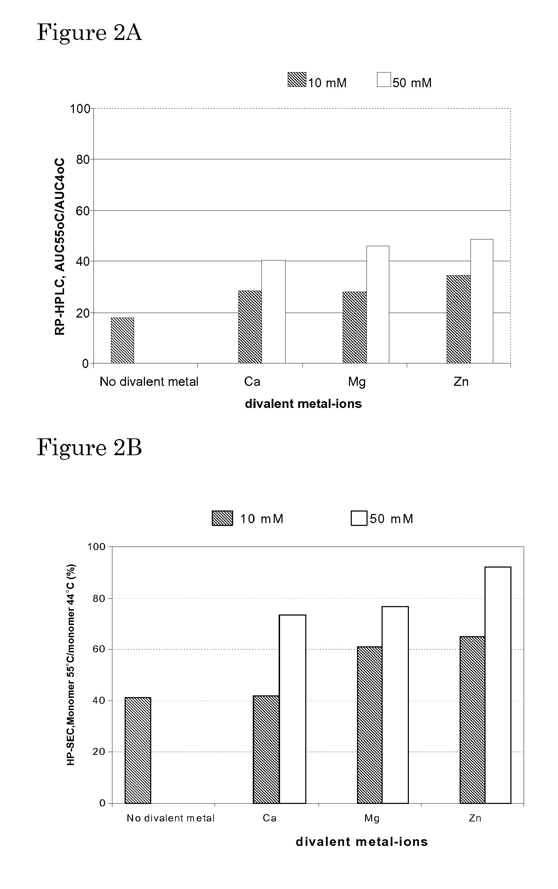

Different divalent metal ions (Ca²⁺, Mg²⁺ or Zn²⁺) were added in concentrations of 0, 10 or 50 mM to formulations comprising oxytocin and 10 mM acetate buffer. All formulations had a final oxytocin-concentration of 0.1 mg/ml and a pH of 4.5. Samples of each formulation were stored for 4 weeks at 4 °C (control) or 55 °C (test). Thereafter, all samples were stored under cooled conditions (2-8 °C) before analysis by RP-HPLC and HP-SEC. Effects on stability were determined by measuring the recovery of oxytocin (i.e. the remaining percentage of oxytocin after incubation at 55 °C compared to the remaining percentage of oxytocin after incubation at 4 °C) and the percentage of oxytocin-monomers. The results are presented in Figures 2A and 2B.

Figure 2A shows the recovery of oxytocin after 4 weeks-storage at 55 °C in acetate buffer in the absence or presence of divalent metal ions. Oxytocin recovery was increased in the presence of divalent metal ions. This effect was observed in a concentration-dependent manner. Figure 2B shows the percentage of remaining oxytocin-monomers after 4 weeks-storage at 55 °C in acetate buffer in the absence or presence of divalent metal ions. Loss of monomers was prevented in the presence of divalent metal ions. These results demonstrate the stabilizing effect of divalent metal ions on a liquid oxytocin formulation.

### EXAMPLE 3

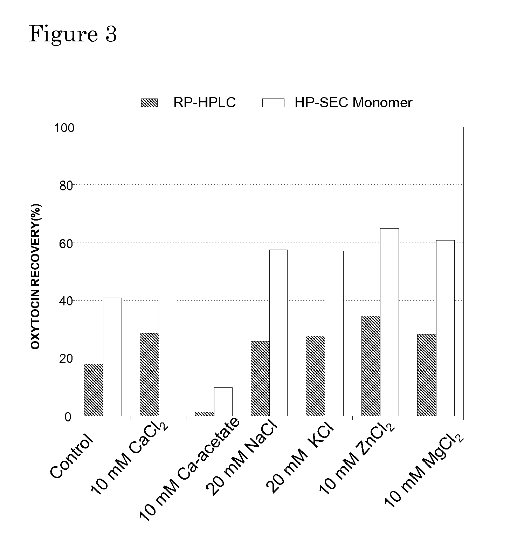

Metal ion sources CaCl₂, Ca-acetate, NaCl, KCl, ZnCl₂ and MgCl₂ were added in concentrations of 0, 10 or 20 mM to formulations comprising oxytocin and 10 mM acetate buffer. All formulations had a final oxytocin-concentration of 0.1 mg/ml and a pH of 4.5. Samples of each formulation were stored for 4 weeks at 4 °C (control) or 55 °C (test). Thereafter, all samples were stored under cooled conditions (2-8 °C) before analysis by RP-HPLC and HP-SEC. Effects on stability were determined by measuring the recovery of oxytocin (i.e. the remaining percentage of oxytocin after incubation at 55 °C compared to the remaining percentage of oxytocin after incubation at 4 °C). The results are presented in Figure 3.

Figure 3 shows the recovery of oxytocin and the remaining percentage of oxytocin-monomers after 4 weeks-storage at 55 °C in acetate buffer in the absence or presence of different metal salts. Oxytocin recovery and the percentage of oxytocin-monomers were increased in the presence of metal chloride salts in a concentration-dependent manner. These results demonstrate the stabilizing effect of metal chloride salts on a liquid oxytocin formulation. Largest increases in stability were observed in the presence of 10 mM ZnCl₂ or 10 mM MgCl₂.

### EXAMPLE 4

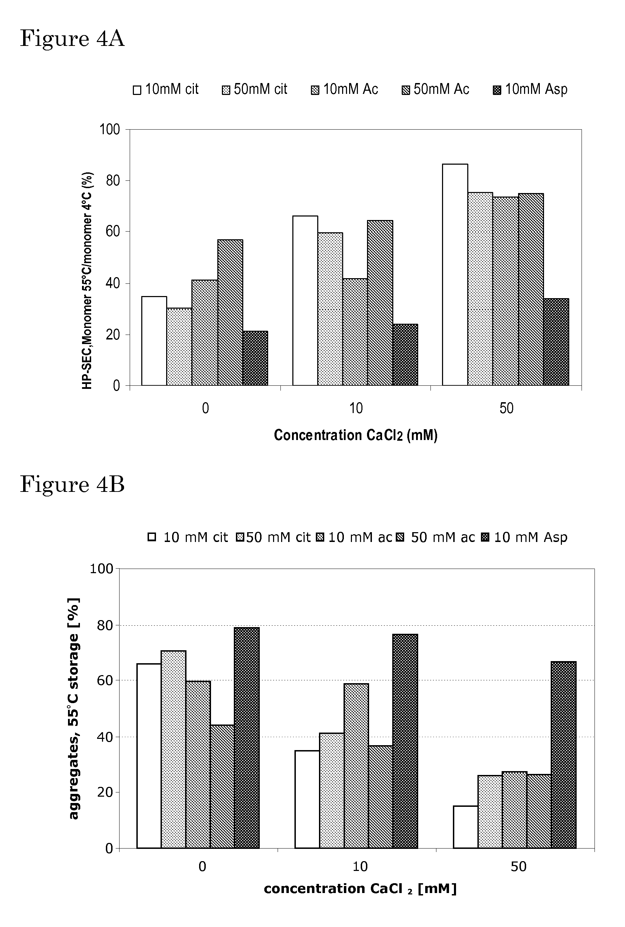

Different buffers were added in concentrations of 10 or 50 mM to formulations comprising oxytocin and 0, 10 or 50 mM CaCl₂. All formulations had a final oxytocin-concentration of 0.1 mg/ml and a pH of 4.5. Samples of each formulation were stored for 4 weeks at 4 °C (control) or 55 °C (test). Thereafter, all samples were stored under cooled conditions (2-8 °C) before analysis by HP-SEC. Effects on stability were determined by measuring the percentage of monomers (i.e. the remaining number of oxytocin monomers after incubation at 55 °C compared to the remaining number of oxytocin monomers after incubation at 4 °C) and the percentage of oxytocin-aggregates (i.e. the number of oxytocin aggregates after incubation at 55 °C compared to the number of oxytocin aggregates after incubation at 4 °C). The results are presented in Figures 4A and 4B.

Figure 4A shows the remaining percentage of oxytocin-monomers after 4 weeks-storage at 55 °C in acetate buffer in the absence or presence of CaCl₂ and different buffers. The remaining percentage of oxytocin-monomers was increased in the presence of CaCl₂ in a concentration-dependent manner. Figure 4B shows the percentage of oxytocin-aggregates. Aggregation was reduced in the presence of CaCl₂ in a concentration-dependent manner. Both the largest remaining percentages and the largest reductions of aggregation were observed in the presence of 50 mM CaCl₂ and 10 or 50 mM citric acid buffer. These results demonstrate the reducing effects of CaCl₂ on oxytocin-aggregation in an aqueous oxytocin formulation.

### EXAMPLE 5

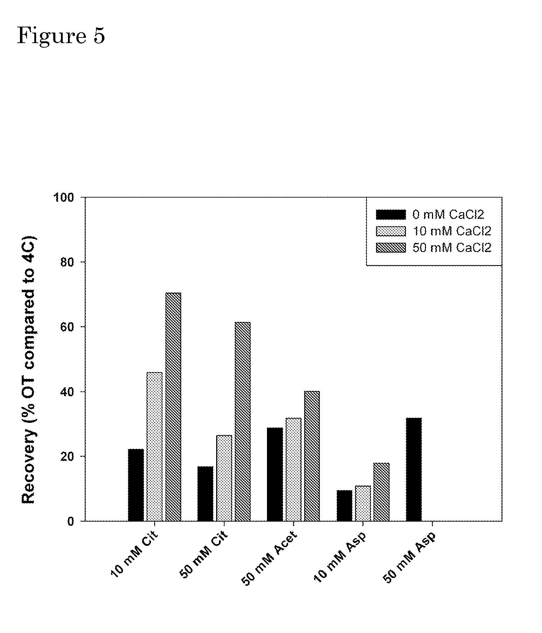

This example exemplifies the oxytocin-stabilizing effect of CaCl₂ in combination with different buffers. Citric acid buffer, acetate buffer and aspartate buffer were added in concentrations of 10 or 50 mM to formulations comprising oxytocin and 0, 10 or 50 mM CaCl₂. All formulations had a final oxytocin-concentration of 0.1 mg/ml and a pH of 4.0. Samples of each formulation were stored for 4 weeks at 4 °C (control) or 55 °C (test). Thereafter, all samples were stored under cooled conditions (2-8 °C) before analysis by RP-HPLC. Effects on stability were determined by measuring the recovery of oxytocin (i.e. the remaining percentage of oxytocin after incubation at 55 °C compared to the remaining percentage of oxytocin after incubation at 4 °C). The results are presented in Figure 5. It demonstrates a concentration-dependent stabilizing effect of CaCl₂, which was enhanced in the presence of citric acid buffer.

### EXAMPLE 6

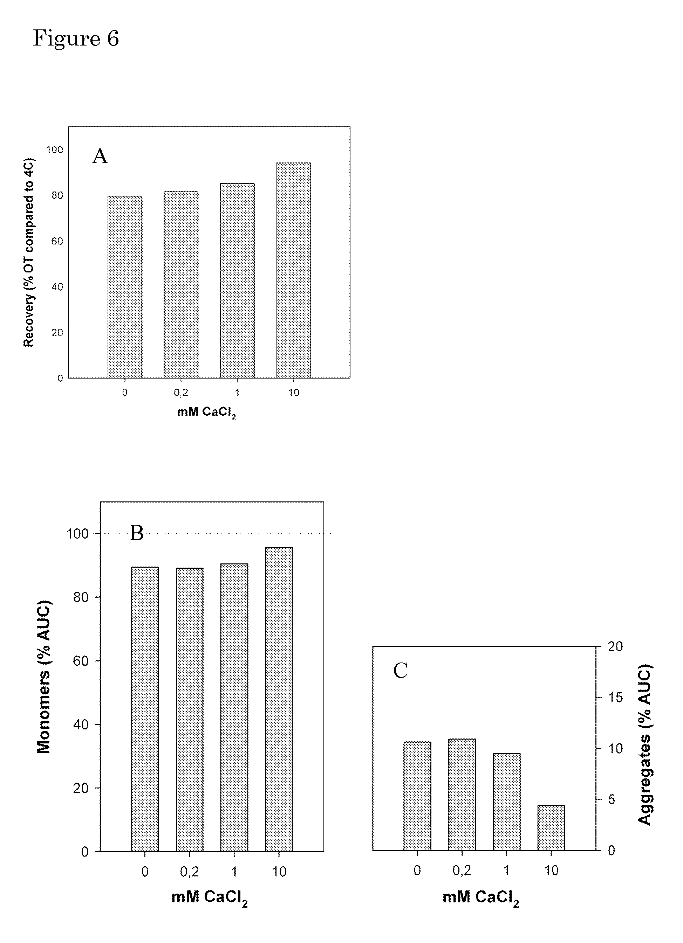

CaCl₂ was added in concentrations of 0, 0.2, 1 or 10 mM to formulations comprising 0.1 mg/ml oxytocin and 10 mM citric acid buffer and having a pH of 4.0. Samples of each formulation were stored for 4 days at 4 °C (control) or 60 °C (test). Thereafter, all samples were stored under cooled conditions (2-8 °C) before analysis by RP-HPLC and HP-SEC. Effects on stability were determined by measuring the recovery of oxytocin (i.e. the remaining percentage of oxytocin after incubation at 60 °C compared to the remaining percentage of oxytocin after incubation at 4 °C), the percentage of oxytocin-monomers and the percentage of formed aggregates in the oxytocin-formulations. The results are presented in Figure 6..

Figure 6A shows the recovery of oxytocin in the presence of different concentrations of CaCl₂ and 10 mM citric acid buffer. In the presence of CaCl₂, oxytocin recovery increased in a concentration-dependent manner.

Figures 6B and 6C show the percentage of oxytocin-monomers and the level of oxytocin-aggregation in the presence different concentrations of CaCl₂ and 10 mM citric acid buffer. In the presence of CaCl₂ and citric acid buffer, the percentage of monomers increased in a concentration-dependent manner. In a similar fashion, the level of oxytocin-aggregation reduced. These results demonstrate the stabilizing effects of CaCl₂ and citric acid buffer.

## Claims

1. An aqueous oxytocin formulation comprising a therapeutically effective amount of oxytocin or a functional analogue thereof, and at least one non-toxic source of metal ions, preferably wherein said source of metal ions is a metal salt.

2. Oxytocin formulation according to claim 1, comprising metal ions in a concentration between 1 and 500 mM, preferably between 5 and 150 mM, more preferably between 10 and 100 mM.

3. Oxytocin formulation according to claim 1 or 2, wherein said metal salt is a metal chloride salt.

4. Oxytocin formulation according to anyone of claims 1 to 3, wherein said metal ions are monovalent metal ions, preferably selected from the group consisting of Na⁺ and K⁺, more preferably wherein said source of metal ions is NaCl or KCl.

5. Oxytocin formulation according to anyone of claims 1 to 4, wherein said metal ions are divalent metal ions, preferably selected from the group consisting of Ca²⁺, Mg²⁺, Cu²⁺ and Zn²⁺, more preferably wherein said source of metal ions is selected from the group consisting of CaCl₂, MgCl₂, CuCl₂ and ZnCl₂.

6. Oxytocin formulation according to claim 5, wherein said source of metal ions is CaCl₂.

7. Oxytocin formulation according to any one of claims 1 to 6, comprising at least one monovalent metal ion and at least one divalent metal ion, preferably comprising at least one monovalent metal chloride salt and / or at least one divalent metal chloride salt.

8. Oxytocin formulation according to any of claims 1 to 7, comprising oxytocin or a functional analogue thereof in a concentration up to 200 IU/ml, preferably up to 100 IU/ml, more preferably between 5 and 100 IU/ml.

9. Oxytocin formulation according to anyone of claims 1 to 8, further comprising a buffer, preferably citric acid, more preferably citric acid in combination with CaCl₂ as metal ion source.

10. Oxytocin formulation according to any of claims 1 to 9, having a pH between 3 and 6, preferably between 3 and 5, more preferably between 3.8 and 4.8.

11. Oxytocin formulation according to any of claims 2 to 10, comprising oxytocin or a functional analogue thereof in a concentration of between 5 and 100 IU/ml, between 5 and 100 mM CaCl₂, between 1 and 100 mM citric acid and wherein the pH of said formulation is between 3.8 and 4.8.

12. Oxytocin formulation according to any of claims 1 to 11 for use as a medication, preferably for the treatment of post-partum haemorrhage.

13. A package, preferably a light resistant package, comprising a formulation according to any of claims 1 to 12 and instructions for use.

14. Use of a formulation according to any of claims 1 to 12 for the manufacture of a medicament for the therapeutic and/or prophylactic treatment of haemorrhage, such as post-partum haemorrhage, preferably wherein said medicament is in the form of a solution, a suspension, an emulsion, a spray or a gel.

15. Use of a formulation according to claim 14, wherein said medicament is formulated for administration via the intravenous, subcutaneous or intramuscular route, the mucosal route, or a combination thereof, preferably wherein said mucosal route is the pulmonary, nasal, sublingual or buccal route.

16. A method for treating or preventing haemorrhage, such as post-partum haemorrhage, in a subject in need thereof, comprising administering to said subject an effective dosage amount of a formulation according to any of claims 1 to 12, preferably wherein said administering comprises administration via the intravenous, subcutaneous or intramuscular route, the mucosal route, or a combination thereof, more preferably wherein said mucosal route is the pulmonary, nasal, sublingual or buccal route.

17. Use of non-toxic metal ions to stabilize an aqueous formulation of oxytocin or a functional analogue thereof.
